# EUROPEAN PATENT APPLICATION

(11) **EP 4 623 964 A1**
(43) Date of publication of application: **01.10.2025**
(21) Application number: 24305449.1
(22) Date of filing: 25.03.2024
(51) Int. Cl.: A61M 5/32

(54) **ANTI-PRICKING SYRINGE NEEDLE SHIELD AND METHOD OF MANUFACTURING THEREOF**

(71) Applicant: Becton Dickinson France, 38800 Le Pont-de-Claix (FR)
(72) Inventor: PERROUD, Corentin, 38500 Voiron (FR); REMPFER, Simon, 38260 St Hilaire de la Cote (FR); DINC, Mirhac, 74960 Annecy (FR); OZTURK, Senturk, 38130 Echirolles (FR); DEVILLARD, Sylvain, 38000 Grenoble (FR)
(74) Representative: Germain Maureau

(57) **Abstract**

Provided herein is a needle shield for use with a syringe comprising a needle and a barrel. The needle shield includes a rigid outer casing forming at least a portion of an outer surface of the needle shield and a compliant inner casing positioned within the rigid outer casing and formed of an elastomeric material, the compliant inner casing having a closed distal end and an open proximal end and defining a cavity therein. The hollow cavity comprises a needle storage portion within which the needle is positioned, with the needle positioned within the needle storage portion such that a sharpened tip of the needle is not embedded in the compliant inner casing and such that the compliant inner casing presses against a circumferential sidewall of the needle, to form a seal between the compliant inner casing and the circumferential sidewall along a portion of a length thereof.

## Description

### BACKGROUND OF THE INVENTION

### Field of the Invention

The present disclosure relates generally to syringes and, more particularly, to a needle shield for a syringe, with the needle shield comprising an anti-pricking needle shield that prevents coring of the needle shield jupe.

### Description of Related Art

Syringes are used in a variety of environments for administering fluids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle and to preserve its sterility prior to use of the injection device.

Needle shields are generally formed to include a rigid component and a soft component or "jupe." The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with a grippable surface for the user to remove the needle shield from the syringe.

While existing needle shields and the soft component therein provide adequate protection to the needle, it is recognized that existing needle shield designs may present drawbacks with regard to performance of the syringe. That is, existing needle shields (and specifically the soft component thereof) can present issues of "coring" of the soft component into the needle - where when the needle shield is applied onto the syringe, the needle is pricked into the soft component of the needle shield to ensure a jupe-needle interface tightness and sealing. However, pricking of the needle into the soft component may cause a coring to occur, where a portion of the rubber (or other elastomeric material) is cut from the soft component and lodged within the lumen of the needle. This core of elastomeric material may thus clog the needle and negatively impact the performing of a subsequent injection using the syringe. Additionally, in some instances, the coring of the soft component by the needle can damage the beveled tip of the needle and/or alter a silicon layer applied onto the beveled tip - with any of these issues potentially impacting penetration of the needle into the patient, such as by increasing a required penetration force or increasing discomfort to a patient during needle penetration.

Accordingly, a need exists in the art for a needle shield that reduces or eliminates the potential for coring to occur between the needle and the soft component of the needle shield.

### SUMMARY OF THE INVENTION

Provided herein is a needle shield for use with a syringe comprising a needle and a barrel. The needle shield includes a rigid outer casing forming at least a portion of an outer surface of the needle shield and a compliant inner casing positioned within the rigid outer casing and formed of an elastomeric material, the compliant inner casing having a closed distal end and an open proximal end and defining a cavity therein. The hollow cavity comprises a needle storage portion within which the needle is positioned when the needle shield is mounted to the barrel, with the needle positioned within the needle storage portion such that a sharpened tip of the needle is not embedded in the compliant inner casing and such that the compliant inner casing presses against a circumferential sidewall of the needle, to form a seal between the compliant inner casing and the circumferential sidewall along a portion of a length of the needle.

In accordance with some aspects of the disclosure, the rigid outer casing comprises a first outer casing half and a second outer casing half.

In accordance with some aspects of the disclosure, the rigid outer casing comprises a vertical split running lengthwise along a portion of a length of the rigid outer casing, the vertical split separating the first outer casing half and the second outer casing half.

In accordance with some aspects of the disclosure, the compliant inner casing is transitionable between an initial configuration and a compressed configuration, with a tip section of the needle storage portion having a first inner diameter that is greater than an outer diameter of the needle when in the initial configuration and a second inner diameter that is equal to the outer diameter of the needle when in the compressed configuration, so as to form the seal between the compliant inner casing and the circumferential sidewall.

In accordance with some aspects of the disclosure, with the vertical split in a closed configuration, the rigid outer casing compresses the compliant inner casing, thereby shrinking the diameter of the tip section to the second inner diameter and pressing the compliant inner casing against the circumferential sidewall of the needle to form the seal therewith.

In accordance with some aspects of the disclosure, the needle shield further includes a compression ring engageable with the rigid outer casing, the compression ring forcing and holding the first outer casing half together with the second outer casing half, to close the vertical split.

In accordance with some aspects of the disclosure, the rigid outer casing comprises an annular groove formed circumferentially about an outer surface thereof, and wherein the compression ring is seated in the annular groove.

In accordance with some aspects of the disclosure, the first outer casing half is ultrasonically welded to the second outer casing half, to close the vertical split.

In accordance with some aspects of the disclosure, the first outer casing half is adhered to the second outer casing half via a glue or adhesive, to close the vertical split.

In accordance with some aspects of the disclosure, the first outer casing half comprises a first clip portion and the second outer casing half comprises a second clip portion, and wherein mating of the first clip portion with the second clip portion closes the vertical split.

Also provided herein is a method of providing a needle shield onto a syringe and over a needle of the syringe. The method includes positioning the needle shield over the needle, such that the needle is received within the needle storage portion in a position where the sharpened tip of the needle is not embedded in the compliant inner casing and the circumferential sidewall of the needle is not in contact with the compliant inner casing. The method also includes positioning the rigid outer casing over the compliant inner casing, with the rigid outer casing in an open configuration where the vertical split therein is open, and transitioning the rigid outer casing from the open configuration to a closed configuration, so as to close the vertical split. In transitioning the rigid outer casing from the open configuration to the closed configuration, the rigid outer casing transitions the compliant inner casing from its initial configuration to its compressed configuration, thereby shrinking the diameter of the tip section from the first inner diameter to the second inner diameter and causing the compliant inner casing to press against the circumferential sidewall of the needle and form the seal between the compliant inner casing and the circumferential sidewall.

In accordance with some aspects of the disclosure, transitioning the rigid outer casing from the open configuration to the closed configuration comprises sliding the compression ring along the rigid outer casing and into engagement with the annular groove, to force the first outer casing half together with the second outer casing half and close the vertical split.

In accordance with some aspects of the disclosure, transitioning the rigid outer casing from the open configuration to the closed configuration comprises: applying an inwardly directed pinching force to the first outer casing half and the second outer casing half, to force the first outer casing half together with the second outer casing half and close the vertical split; and bonding the first outer casing half together with the second outer casing half to keep the vertical split closed.

In accordance with some aspects of the disclosure, bonding the first outer casing half together with the second outer casing half comprises ultrasonically welding the first outer casing half to the second outer casing half along a length of the vertical split.

In accordance with some aspects of the disclosure, bonding the first outer casing half together with the second outer casing half comprises adhering the first outer casing half to the second outer casing half along a length of the vertical split, via an adhesive or glue.

Also provided herein is a prefilled or prefillable syringe. The syringe comprises a syringe assembly including a barrel having a barrel distal end and a barrel proximal end and defining a chamber, the barrel including a barrel end portion at the barrel distal end, a needle having a needle tip at a distal end of the needle and having a proximal end fixedly inserted into the barrel end portion, and the needle shield mounted to the barrel via the barrel end portion, so as to be positioned over the needle. The syringe also comprises a plunger received within the chamber of the barrel and axially movable therein. The needle shield of the syringe assembly further includes a rigid outer casing forming at least a portion of an outer surface of the needle shield and a compliant inner casing positioned within the rigid outer casing and formed of an elastomeric material, the compliant inner casing having a closed distal end and an open proximal end and defining a cavity therein. The hollow cavity comprises a needle storage portion within which the needle is positioned when the needle shield is mounted to the barrel, with the needle positioned within the needle storage portion such that a sharpened tip of the needle is not embedded in the compliant inner casing and such that the compliant inner casing presses against a circumferential sidewall of the needle, to form a seal between the compliant inner casing and the circumferential sidewall along a portion of a length of the needle.

### BRIEF DESCRIPTION OF THE DRAWINGS

FIG. 1 is a perspective view of a syringe including a needle shield, according to an embodiment of the disclosure;
FIG. 2 is an exploded view of the syringe of FIG. 1;
FIG. 3 is a cross-sectional view of a needle shield that may be included on the syringe of FIG. 1, according to an embodiment of the disclosure;
FIG. 4A is an isolated view of a compliant inner casing of the needle shield of FIG. 3 relative to the syringe needle, with the compliant inner casing in an initial configuration;
FIG. 4B is an isolated view of a compliant inner casing of the needle shield of FIG. 3 relative to the syringe needle, with the compliant inner casing in a compressed configuration;
FIG. 5A is a side view of a needle shield that may be included on the syringe of FIG. 1, with a rigid outer casing thereof in an open configuration, according to an embodiment of the disclosure;
FIG. 5B is a side view of the needle shield of FIG. 5A in a closed configuration;
FIG. 6A is a side view of a needle shield that may be included on the syringe of FIG. 1, with a rigid outer casing thereof in an open configuration, according to another embodiment of the disclosure;
FIG. 6B is a side view of the needle shield of FIG. 6A, with a having a pinching force applied thereto; and
FIG. 6C is a side view of the needle shield of FIG. 6A in a closed configuration;
FIG. 7A is a side view of a needle shield that may be included on the syringe of FIG. 1, with a rigid outer casing thereof in an open configuration, according to another embodiment of the disclosure; and
FIG. 7B is a side view of the needle shield of FIG. 7A in a closed configuration.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

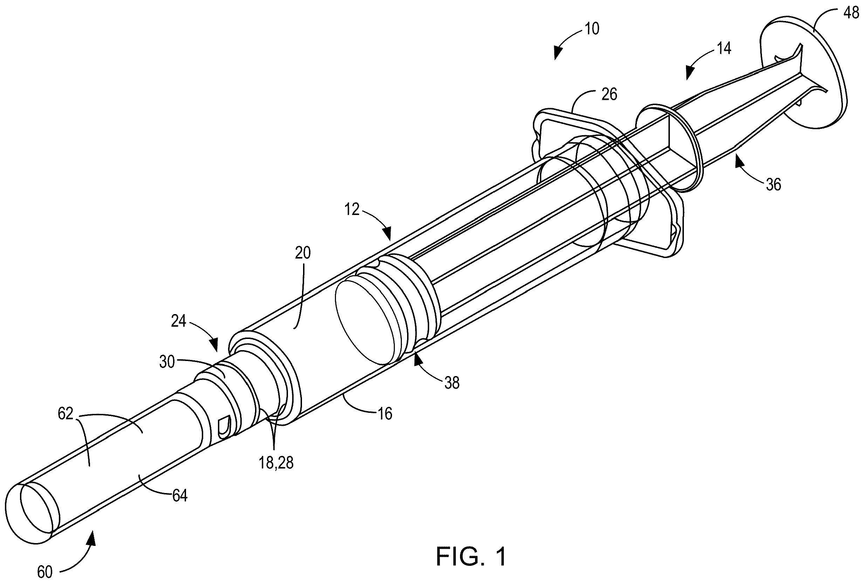

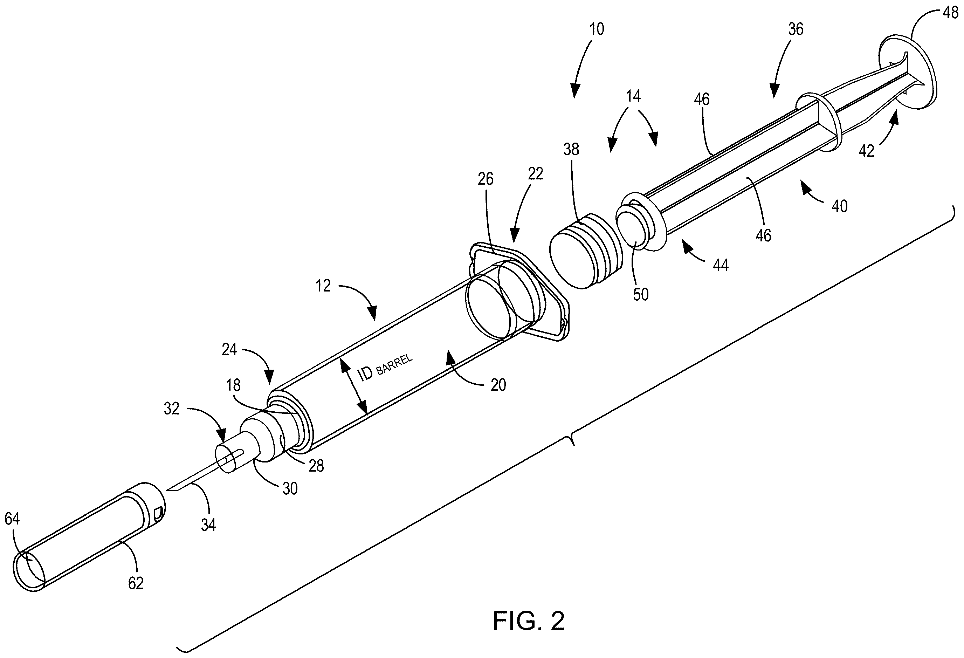

Referring to FIGS. 1 and 2, shown is a non-limiting embodiment of a medical injection device 10, such as a syringe for example, with which aspects or embodiments of the disclosure may be implemented. While the medical injection device is shown and described hereafter as a syringe 10, it is recognized that other medical injection devices may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, with a proximal end of the needle 34 positioned within the channel 32 and a distal end of the needle 34 extending out from the hub portion 30. The proximal end of the needle 34 may be inserted into the channel 32 of hub portion 30 and may be fixed therein with an adhesive (e.g., glue), by thermal welding, or the like. The needle 34 defines a hollow lumen therein that is in fluid communication with the chamber 20 of syringe barrel 12, such that fluid may be dispensed from the syringe 10.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

As shown in FIGS. 1 and 2, a needle shield 60 of syringe assembly 12 is coupled to the syringe barrel 12 to protect the needle 34. According to aspects of the disclosure, the needle shield 60 is configured to be mounted to the barrel 12 via coupling of the needle shield 60 to the hub 30 of the barrel 16. As explained in further detail below, the needle shield 60 may be composed of a rigid material or semi-rigid material that provides structure to the needle shield 60, as well as an elastomeric material (i.e., a deformable or compliant material) that surrounds the needle 34 when the needle shield 60 is coupled to the syringe barrel 12 and may also present a mating surface for the hub 30 of barrel 12. In various embodiments, the rigid material may be a resin such as polypropylene (PP), polyethylene (PE), polyethylene terephthalate (PET), polystyrene (PS), polycarbonate (PC), or polyvinyl chloride (PVC), as non-limiting examples, while the elastomeric material may be an elastic rubber (e.g., isoprene rubber, butyl rubber, latex rubber, silicone rubber or the like) or a thermoplastic elastomer (TPE), as non-limiting examples.

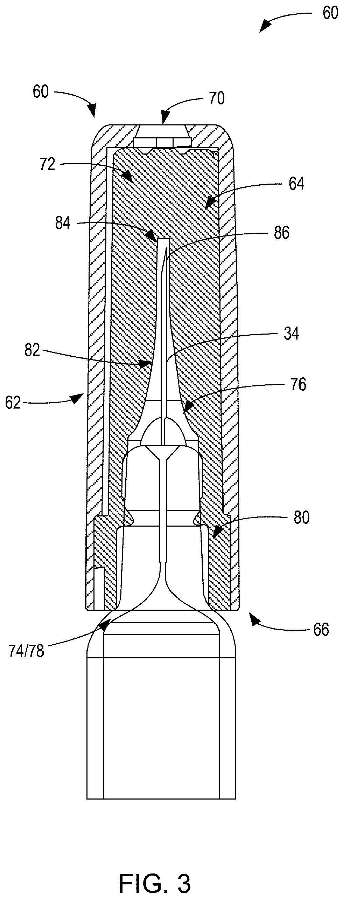

As shown best in FIG. 3, the needle shield 60 is composed of a rigid outer casing 62 that provides structure to the needle shield 60 and a compliant inner casing or jupe 64 that surrounds the needle 34 (when needle shield 60 is coupled to syringe barrel 12).

The rigid outer casing 62 may be constructed as a generally tubular member that includes an open proximal end portion 66 and a mostly closed distal end portion 68. The distal end portion 68 may include an opening 70 therein having a smaller diameter than a diameter of the compliant inner casing 64, such that the compliant inner casing 64 may be better retained in the rigid outer casing 62. The compliant inner casing 64 is formed as an elongated cylindrical body that is sized to be positioned within the outer casing 62, so as to be retained therein. The compliant inner casing 64 may be characterized as having a closed distal end portion 72 and an open proximal end portion 74. A hollow cavity 76 is formed in the compliant inner casing 64, with an opening 78 in the compliant inner casing 64 providing access into the cavity 76. The cavity 76 extends distally into the compliant inner casing 64 a portion of the length thereof.

According to aspects of the disclosure, the hollow cavity 76 may be characterized as including a barrel end storage portion 80 and a needle storage portion 82. The open proximal end 74 (i.e., opening 80 therein) allows for positioning of the needle shield 60 onto the hub 30 of syringe barrel 12. The hub 30 may be introduced through open proximal end 74 and positioned within the barrel end storage portion 80, with the hub 30 sized to form a tight fit within the barrel end storage portion 80 to ensure engagement of the needle shield 60 to the barrel 12.

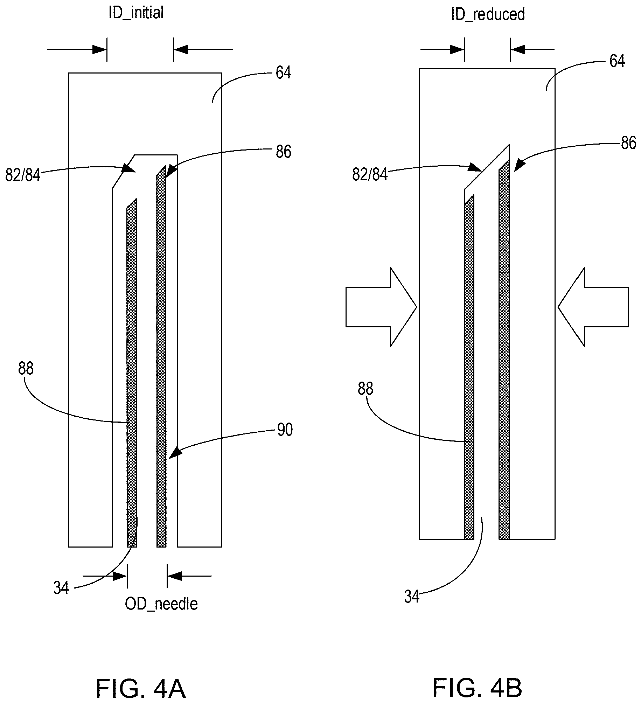

The needle storage portion 82 is sized to receive the needle 34 therein, to provide protection to the needle 34 once the needle shield 60 is coupled to barrel 12. According to embodiments of the disclosure, the needle storage portion 82 has an inner diameter that tapers as the needle storage portion 82 extends from its proximal end to its distal end. In some embodiments, the proximal end of the needle storage portion 82 may be formed as a tapered portion having a diameter that reduces proximally to distally to prevent the insertion of the needle 34 into the compliant inner casing 64 and securely guide the needle 34 distally. The distal end of the needle storage portion 82 is formed with a small diameter tip section 84 having an initial inner diameter, ID_initial, slightly larger than the outer diameter of the needle 34, OD_needle, with the tip section 84 having a constant inner diameter, as shown in FIGS. 4A and 4B.

According to embodiments of the disclosure, the hollow cavity 76 of needle shield 60 is configured such that, when the hub 30 of the barrel 12 is inserted into the needle shield 60 and is received in the barrel end storage portion 80 of the needle shield 60, a sharpened tip 86 of the needle 34 enters the small diameter tip section 84 of the needle storage portion 82 but does not protrude therethrough so as to reach the compliant material of closed distal end 72 that is positioned distally from the small diameter tip section 84 of the needle storage portion 82 - i.e., the sharpened tip 86 does not puncture and extend into compliant inner casing 64. Accordingly, contact between the sharpened tip 86 and the compliant inner casing 64 is reduced or eliminated, and this elimination/reduction in contact between the sharpened tip 86 and the compliant inner casing 64 may serve to prevent "coring" of the compliant inner casing 64 and/or damaging of the sharpened tip 86 (e.g., damaging the beveling of tip 86 when needle 34 is embedded in the compliant inner casing 64 and/or damaging of a siliconized layer on the tip 86 when needle 34 is removed from the compliant inner casing 64) that might occur if the sharpened tip 86 were pricked into the compliant inner casing 64.

According to aspects of the disclosure, it is recognized that if the sharpened tip 86 is not pricked into the compliant inner casing 64 to form a jupe-needle interface tightness and sealing (to prevent liquid from escaping the needle 34), then it is desirable to still form a seal about the needle 34 in some fashion. To provide such sealing, the needle shield 60 may be configured to enable a compression and deformation of the compliant inner casing 64 in a manner that shrinks the diameter of the tip section 84 (of needle storage portion 82) and brings the compliant inner casing 64 into contact with a cylindrical sidewall 88 of the needle 34, along at least a portion of a length thereof. That is, as illustrated in FIGS. 4A and 4B, it is desirable to compress the compliant inner casing 64 from an initial configuration to a compressed configuration, whereby an initial inner diameter, ID_initial, of the small diameter tip section 84 is shrunk to a reduced diameter, ID_reduced. With the compression of the compliant inner casing 64 from its initial configuration to its compressed configuration, a gap 90 that is initially present between the compliant inner casing 64 and the cylindrical sidewall 88 of the needle 34 (in the area of tip section 84) is eliminated, and the compliant inner casing 64 is brought into contact with the cylindrical sidewall 88 of the needle 34, so as to form a seal between the compliant inner casing 64 and the cylindrical sidewall 88 along a portion of a length of the needle 34.

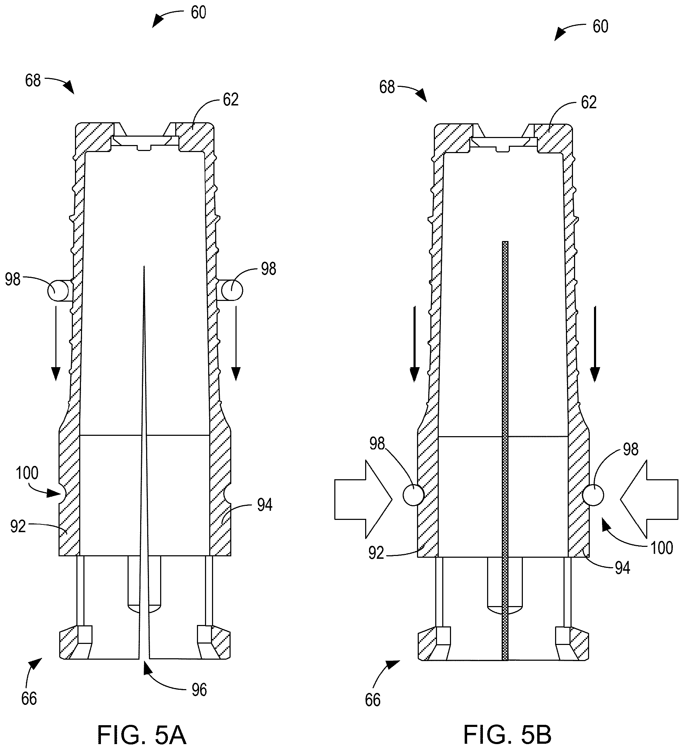

Referring now to FIGS. 5-7, in order to provide for a transitioning of the compliant inner casing 64 from its initial configuration to its compressed configuration, the rigid outer casing 62 is specifically configured to provide a radially inward pressure against the compliant inner casing 64, when the compliant inner casing 64 is secured within the rigid outer casing 62. Specifically, the rigid outer casing 62 is formed of a first outer casing half 92 and a second outer casing half 94 whose positioning relative to each other may be changed, to selectively apply such pressure to the compliant inner casing 64. In accordance with aspects of the disclosure, the first outer casing half 92 and the second outer casing half 94 may be joined along a portion of a length of the rigid outer casing 62 - i.e., joined at the distal end portion 68 of the rigid outer casing 62 - but a vertical split 96 is formed in the rigid outer casing 62 at the proximal end portion 66 that runs lengthwise along a portion of a length of the rigid outer casing 62 to separate the first outer casing half 92 from the second outer casing half 94 at the proximal end portion 66.

According to aspects of the disclosure, the vertical split 96 may be transitioned from an open configuration to a closed configuration, in order to selectively provide compression to the compliant inner casing 64 when positioned within the rigid outer casing 62. That is, with the vertical split 96 being in the open configuration, the compliant inner casing 64 may be retained within the rigid outer casing 62 without any radially inward-directed pressure being applied against the compliant inner casing 64 by the outer casing 62 - such that the compliant inner casing 64 remains in its initial configuration. However, when the first and second outer casing halves 92, 94 are pressed together and the vertical split 96 is caused to transition to the closed configuration, the rigid outer casing 62 then applies a radially inward-directed pressure against the compliant inner casing 64 (due to an inner diameter of the rigid outer casing 62 being reduced), which compresses the compliant inner casing 64 and transitions the compliant inner casing 64 to its compressed configuration. As described above, when the compliant inner casing 64 transitions to its compressed configuration, a gap 90 that is initially present between the compliant inner casing 64 and the cylindrical sidewall 88 of the needle 34 in the area of tip section 84 (FIG. 4A) is eliminated, and the compliant inner casing 64 is brought into contact with the cylindrical sidewall 88 of the needle 34 (FIG. 4B), so as to form a seal between the compliant inner casing 64 and the cylindrical sidewall 88 along a portion of a length of the needle 34.

According to aspects of the disclosure, a closing of the vertical split 96 from its open configuration to its closed configuration - and a retaining of the vertical split 96 in its closed configuration - may be achieved via a number of different means.

As shown in FIGS. 5A and 5B, according to one embodiment of the disclosure, the needle shield 60 may further include a compression ring 98 that is engageable with the rigid outer casing 62 to selectively close the vertical split 96 when secured thereon. In some embodiments, the compression ring 98 may be slid from the distal end portion 68 of the rigid outer casing 62 (FIG. 5A) down toward the proximal end portion 66 (FIG. 5B), in order to close the vertical split 96. The distal end portion 68 of the rigid outer casing 62 may have a reduced diameter as compared to the proximal end portion 66, such that when the compression ring 98 is slid down onto the proximal end portion 66 and engages an annular groove 100 formed circumferentially about an outer surface of the proximal end portion 66 (within which the compression ring 98 may be seated), the compression ring 98 applies a radially inward-directed pressure. The radially inward-directed pressure applied by the compression ring 98 closes the vertical split 96 between the first outer casing half 92 and the second outer casing half 94, thus reducing an inner diameter of the rigid outer casing 62. This reduction in the diameter of the rigid outer casing 62 causes a compressive force to be applied to the compliant inner casing 64 that is retained therein, which as explained above, transitions the compliant inner casing 64 to its compressed configuration and brings the compliant inner casing 64 into contact with the cylindrical sidewall 88 of the needle 34 (FIG. 4B) in the tip section 84 of the needle storage portion 82 of cavity 76. A seal is thus formed between the compliant inner casing 64 and the cylindrical sidewall 88 along a portion of a length of the needle 34.

Accordingly, a method of providing the needle shield 60 onto syringe 10 and over needle 34 may include positioning the compliant inner casing 64 over the needle 34 such that the needle 34 is received within the needle storage portion 82 in a position where the sharpened tip 86 of the needle 34 is not embedded in the compliant inner casing 64 and the cylindrical sidewall 88 of the needle 34 is not in contact with the compliant inner casing 64. The method also includes positioning the rigid outer casing 62 over the compliant inner casing 64, with the rigid outer casing 62 in an open configuration where the vertical split 96 therein is open, and then transitioning the rigid outer casing 62 from the open configuration to a closed configuration, so as to close the vertical split 96. In transitioning the rigid outer casing 62 from the open configuration to the closed configuration, the compression ring 98 is slid proximally along the rigid outer casing 62 and into engagement with the annular groove 100, to force the first outer casing half 92 together with the second outer casing half 94 and close the vertical split 96. Closing of the vertical split 96 transitions the compliant inner casing 64 from its initial configuration to its compressed configuration, thereby shrinking the diameter of the needle storage portion 82 (i.e., of tip section 84 thereof) from the first inner diameter to the second inner diameter and causing the compliant inner casing 64 to press against the cylindrical sidewall 88 of the needle 34 and form the seal between the compliant inner casing 64 and the cylindrical sidewall 88.

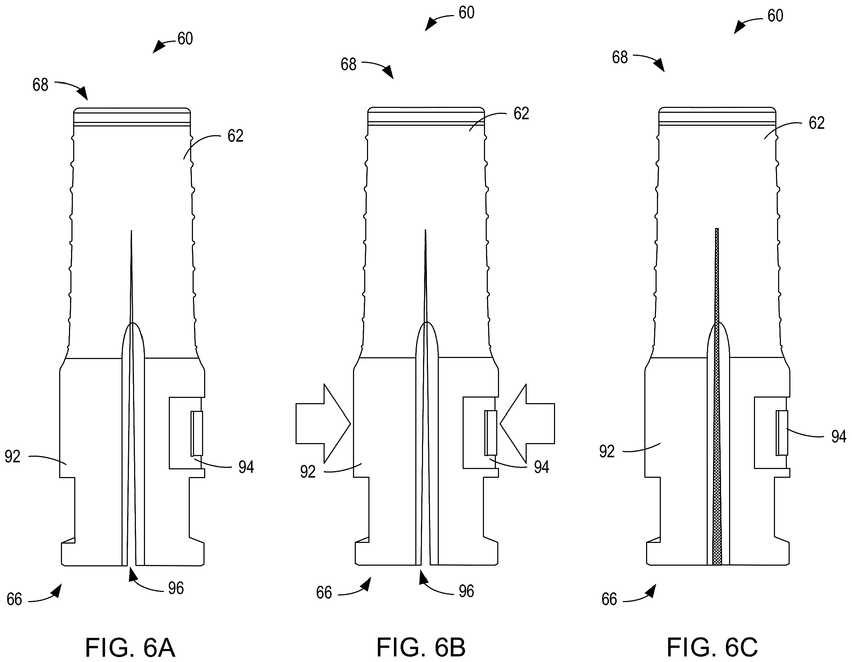

As shown in FIGS. 6A and 6B, according to another embodiment of the disclosure, the vertical split 96 in the rigid outer casing 62 may be closed via combination of a pinching force being applied to the first outer casing half 92 and the second outer casing half 94 and a subsequent bonding of the first and second outer casing halves 92, 94 together. According to embodiments, a pinching force may be applied to the first outer casing half 92 and the second outer casing half 94 (FIG. 6B), such as via a mechanized apparatus, and upon closing of the vertical split 96, the first and second outer casing halves 92, 94 may then be bonded together (FIG. 6C), such as via welding the first outer casing half 92 to the second outer casing half 94 (e.g., ultrasonic welding or plastic welding, as non-limiting example) or gluing (or otherwise adhering) the first outer casing half 92 to the second outer casing half 94, as non-limiting examples. As described above, with the vertical split 96 between the first outer casing half 92 and the second outer casing half 94 closed, an inner diameter of the rigid outer casing 62 is reduced, and this reduction in the diameter of the rigid outer casing 62 causes a compressive force to be applied to the compliant inner casing 64 that is retained therein - which transitions the compliant inner casing 64 to its compressed configuration and brings the compliant inner casing 64 into contact with the cylindrical sidewall 88 of the needle 34 (FIG. 4B) in the tip section 84 of the needle storage portion 82 of cavity 76. A seal is thus formed between the compliant inner casing 64 and the cylindrical sidewall 88 along a portion of a length of the needle 34.

Accordingly, a method of providing the needle shield 60 onto syringe 10 and over needle 34 may include positioning the compliant inner casing 64 over the needle 34 such that the needle 34 is received within the needle storage portion 82 in a position where the sharpened tip 86 of the needle 34 is not embedded in the compliant inner casing 64 and the cylindrical sidewall 88 of the needle 34 is not in contact with the compliant inner casing 64. The method also includes positioning the rigid outer casing 62 over the compliant inner casing 64, with the rigid outer casing 62 in an open configuration where the vertical split 96 therein is open, and then transitioning the rigid outer casing 62 from the open configuration to a closed configuration, so as to close the vertical split 96. In transitioning the rigid outer casing 62 from the open configuration to the closed configuration, an inwardly directed pinching force may be applied to the first outer casing half 92 and the second outer casing half 94, to force the first outer casing half 92 together with the second outer casing half 94 and close the vertical split 96, and subsequently bonding the first outer casing half 92 together with the second outer casing half 94 to keep the vertical split 96 closed. Bonding of the first outer casing half 92 together with the second outer casing half 94 may comprise welding (ultrasonic welding or plastic welding) the first outer casing half 92 to the second outer casing half 94 along a length of the vertical split 96 or adhering the first outer casing half 92 to the second outer casing half 94 along a length of the vertical split 96, via an adhesive or glue. Closing of the vertical split 96 transitions the compliant inner casing 64 from its initial configuration to its compressed configuration, thereby shrinking the diameter of the needle storage portion 82 (i.e., of tip section 84 thereof) from the first inner diameter to the second inner diameter and causing the compliant inner casing 64 to press against the cylindrical sidewall 88 of the needle 34 and form the seal between the compliant inner casing 64 and the cylindrical sidewall 88.

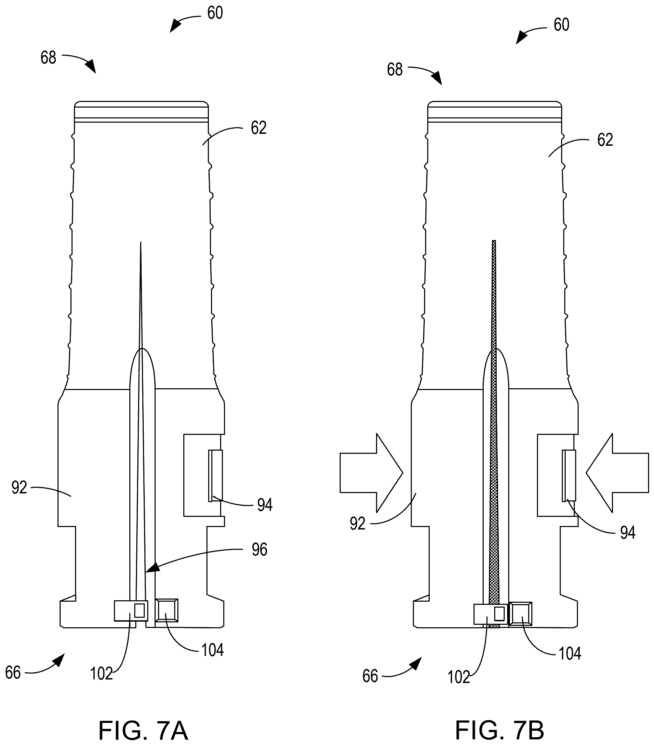

As shown in FIGS. 7A and 7B, according to one embodiment of the disclosure, the needle shield 60 may further include mechanical connectors on the rigid outer casing 62 that close the vertical split 96 when joined together. In some embodiments, the mechanical connectors may be provided as a clip 102 (a first clip portion) formed on the first outer casing half 92 and a corresponding receptacle 104 (a second clip portion) formed on the second outer casing half 94. When a pinching force is applied to the first and second outer casing halves 92, 94 that push the halves together and closes the vertical split 96, the clip 102 may engage the receptacle 104. With the clip 102 engaged with and retained in the receptacle 104, the vertical split 96 is kept closed, thus reducing an inner diameter of the rigid outer casing 62. This reduction in the diameter of the rigid outer casing 62 causes a compressive force to be applied to the compliant inner casing 64 that is retained therein, which as explained above, transitions the compliant inner casing 64 to its compressed configuration and brings the compliant inner casing 64 into contact with the cylindrical sidewall 88 of the needle 34 (FIG. 4B) in the tip section 84 of the needle storage portion 82 of cavity 76. A seal is thus formed between the compliant inner casing 64 and the cylindrical sidewall 88 along a portion of a length of the needle 34.

Accordingly, a method of providing the needle shield 60 onto syringe 10 and over needle 34 may include positioning the compliant inner casing 64 over the needle 34 such that the needle 34 is received within the needle storage portion 82 in a position where the sharpened tip 86 of the needle 34 is not embedded in the compliant inner casing 64 and the cylindrical sidewall 88 of the needle 34 is not in contact with the compliant inner casing 64. The method also includes positioning the rigid outer casing 62 over the compliant inner casing 64, with the rigid outer casing 62 in an open configuration where the vertical split 96 therein is open, and then transitioning the rigid outer casing 62 from the open configuration to a closed configuration, so as to close the vertical split 96. In transitioning the rigid outer casing 62 from the open configuration to the closed configuration, an inwardly directed pinching force may be applied to the first outer casing half 92 and the second outer casing half 94, to force the first outer casing half 92 together with the second outer casing half 94 and close the vertical split 96, with the clip 102 on the first outer casing half 92 engaging the receptacle 104 on the second outer casing half 94 to keep the vertical split 96 closed. Closing of the vertical split 96 transitions the compliant inner casing 64 from its initial configuration to its compressed configuration, thereby shrinking the diameter of the needle storage portion 82 (i.e., of tip section 84 thereof) from the first inner diameter to the second inner diameter and causing the compliant inner casing 64 to press against the cylindrical sidewall 88 of the needle 34 and form the seal between the compliant inner casing 64 and the cylindrical sidewall 88.

Beneficially, embodiments of the disclosure provide a needle shield that reduces or eliminates the potential for coring to occur between the needle and the soft component of the needle shield. A sharpened tip of the needle is positioned within a needle storage portion of a cavity formed in the compliant inner casing of the needle shield, so as not to be embedded within the compliant inner casing. The compliant inner casing may be compressed by a rigid outer casing of the needle shield, to bring the compliant inner casing into contact with a circumferential sidewall of the needle, to form a seal between the compliant inner casing and the circumferential sidewall along a portion of a length of the needle.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A needle shield for use with a syringe comprising a needle and a barrel, the needle shield comprising:
a rigid outer casing forming at least a portion of an outer surface of the needle shield; and
a compliant inner casing positioned within the rigid outer casing and formed of an elastomeric material, the compliant inner casing having a closed distal end and an open proximal end and defining a cavity therein;
wherein the hollow cavity comprises a needle storage portion within which the needle is positioned when the needle shield is mounted to the barrel, with the needle positioned within the needle storage portion such that a sharpened tip of the needle is not embedded in the compliant inner casing and such that the compliant inner casing presses against a circumferential sidewall of the needle, to form a seal between the compliant inner casing and the circumferential sidewall along a portion of a length of the needle.

2. The needle shield of claim 1, wherein the rigid outer casing comprises a first outer casing half and a second outer casing half.

3. The needle shield of claim 2, wherein the rigid outer casing comprises a vertical split running lengthwise along a portion of a length of the rigid outer casing, the vertical split separating the first outer casing half and the second outer casing half.

4. The needle shield of claim 2, wherein the compliant inner casing is transitionable between an initial configuration and a compressed configuration, with a tip section of the needle storage portion having a first inner diameter that is greater than an outer diameter of the needle when in the initial configuration and a second inner diameter that is equal to the outer diameter of the needle when in the compressed configuration, so as to form the seal between the compliant inner casing and the circumferential sidewall.

5. The needle shield of claim 4, wherein with the vertical split in a closed configuration, the rigid outer casing compresses the compliant inner casing, thereby shrinking the diameter of the tip section to the second inner diameter and pressing the compliant inner casing against the circumferential sidewall of the needle to form the seal therewith.

6. The needle shield of claim 5, further comprising a compression ring engageable with the rigid outer casing, the compression ring forcing and holding the first outer casing half together with the second outer casing half, to close the vertical split.

7. The needle shield of claim 6, wherein the rigid outer casing comprises an annular groove formed circumferentially about an outer surface thereof, and wherein the compression ring is seated in the annular groove.

8. The needle shield of claim 5, wherein the first outer casing half is welded to the second outer casing half, to close the vertical split.

9. The needle shield of claim 5, wherein the first outer casing half is adhered to the second outer casing half via a glue or adhesive, to close the vertical split.

10. The needle shield of claim 5, wherein the first outer casing half comprises a first clip portion and the second outer casing half comprises a second clip portion, and wherein mating of the first clip portion with the second clip portion closes the vertical split.

11. A method of providing the needle shield of claims 1-10 onto a syringe and over a needle of the syringe, the method comprising:
positioning the needle shield over the needle, such that the needle is received within the needle storage portion in a position where the sharpened tip of the needle is not embedded in the compliant inner casing and the circumferential sidewall of the needle is not in contact with the compliant inner casing;
positioning the rigid outer casing over the compliant inner casing, with the rigid outer casing in an open configuration where the vertical split therein is open; and
transitioning the rigid outer casing from the open configuration to a closed configuration, so as to close the vertical split;
wherein in transitioning the rigid outer casing from the open configuration to the closed configuration, the rigid outer casing transitions the compliant inner casing from its initial configuration to its compressed configuration, thereby shrinking the diameter of the tip section of the needle storage portion from the first inner diameter to the second inner diameter and causing the compliant inner casing to press against the circumferential sidewall of the needle and form the seal between the compliant inner casing and the circumferential sidewall.

12. The method of claim 11, wherein transitioning the rigid outer casing from the open configuration to the closed configuration comprises sliding the compression ring along the rigid outer casing and into engagement with the annular groove, to force the first outer casing half together with the second outer casing half and close the vertical split.

13. The method of claim 11, wherein transitioning the rigid outer casing from the open configuration to the closed configuration comprises:
applying an inwardly directed pinching force to the first outer casing half and the second outer casing half, to force the first outer casing half together with the second outer casing half and close the vertical split; and
bonding the first outer casing half together with the second outer casing half to keep the vertical split closed.

14. The method of claim 13, wherein bonding the first outer casing half together with the second outer casing half comprises one of welding the first outer casing half to the second outer casing half along a length of the vertical split, and adhering the first outer casing half to the second outer casing half along a length of the vertical split, via an adhesive or glue.

15. A prefilled or prefillable syringe, the syringe comprising:
a syringe assembly including:
a barrel having a barrel distal end and a barrel proximal end and defining a chamber, the barrel including a barrel end portion at the barrel distal end;
a needle having a needle tip at a distal end of the needle and having a proximal end fixedly inserted into the barrel end portion; and
the needle shield of claims 1-10, with the needle shield mounted to the barrel via the barrel end portion, so as to be positioned over the needle; and
a plunger received within the chamber of the barrel and axially movable therein.
